# EUROPEAN PATENT APPLICATION

(11) **EP 1 279 961 A2**
(43) Date of publication of application: **29.01.2003**
(21) Application number: 02255265.7
(22) Date of filing: 23.07.2002
(51) Int. Cl.: G01N 33/573, G01N 33/543, G01N 33/58

(54) **Non-isotopic method and kit for quantifying enzyme inhibitors**

(30) Priority: 23.07.2001 US 307570
(71) Applicant: Norzyme, Inc., Aliso Viejo, California 92656 (US)
(72) Inventor: Gan, Zhibo, Aliso Viejo, California 92656 (US); Wallman, Jeanette, Aliso Viejo, California 92656 (US)
(74) Representative: Waddington, Richard

(57) **Abstract**

A method, kit, and assay for measuring levels of a therapeutic compound in a sample by providing a reaction vessel containing a sample, the sample includes a therapeutic compound having a biological activity and biological substance. A probe coated with a substrate for the biological substance is provided, the substrate having a detectable label. The probe is inserted into the reaction vessel such that the therapeutic compound and the substrate contact the biological substance and interact within the biological substance resulting in the label being released. The probe is removed from the reaction vessel and the quantity of detectable label is measured, thereby measuring the level of therapeutic compound. The concentration of therapeutic compound is calculated based upon a standard curve or by calculating the percentage of inhibition and expressing the results in arbitrary units.

## Description

### CROSS-REFERENCE TO RELATED APPLICATIONS

This application claims the benefit of priority under 35 U.S.C. Section 119(e) of United States Provisional Patent Application No. 60/307,570, filed July 23, 2001, which is incorporated herein by reference.

### BACKGROUND OF THE INVENTION

### 1. FIELD OF THE INVENTION

The present invention relates generally to the field of infection treatment and to methods and devices for measuring, monitoring, and detecting the identity, amount, and activity of therapeutic compounds. More specifically, the present invention relates to a method and a device for measuring levels of protease inhibitors in blood.

### 2. DESCRIPTION OF THE RELATED ART

Enzymes play a key role in biochemical reactions. The determination of their activity is important in all fields related to biology, such as medicine, food, and pharmacy. The methods currently used for enzyme assays are mainly based on the formation of product from substrate following enzyme catalysis (Loround, 1981; Rossomando, 1990). Although these methods are the mainstream of much biological research, there is a need to determine the activity of an enzyme, the quantity of the enzyme present, and the identity of the enzyme.

As virtually all chemical reactions in living systems are catalyzed by enzymes, the assay of enzyme activity is one of the most frequently encountered procedures in biochemistry. Typically, most enzyme assays are used to estimate the amount or activity of an enzyme present in a cell, tissue, other preparation, or as an essential part of an enzyme purification protocol. Current assay methods have been developed based on the physical, chemical, and immunological properties of the enzyme of interest and use detection means such as photometric, radiometric, high performance liquid chromatographic, and electrochemical assays. (Eisenthal, R. and Danson, M. J., in *Enzyme Assays: a practical approach*. (IRL Press: Oxford University, Oxford, (1993)). Although the above-described methods basically fulfill the requirements for routine analysis, there are the disadvantages of low sensitivity (Oppert et al, 1997, *Bio Techniques* 23:70-72), multiple steps (Twining, S. S., 1984 *Anal Biochem* 143: 30-344; Pazhanisamy, S. et al, 1995, *Anal Biochem* 229: 48-53), and steps that are tedious and time-consuming (Fields, R.,. 1972, *Meth Enz* 25B: 464-468).

Similarly, immunoassays have been widely used in human clinical tests and therapeutics, agriculture, food, veterinary and environmental diagnostics (Deshpandes, S. in *Enzyme immunoassays from concept to product development.,* (Chapman & Hall: New York, 1996)). While for most purposes immunoassays are effective (Cleaveland, J. S. et al, 1990, *Anal Biochem* 190: 249-253.), in some cases they are not suitable, such as in the determination of enzyme activity. This is due to the fact that binding assays for antibody and antigen (in this instance, an enzyme) only measure the concentration of an antigen (enzyme) and not its activity. It is often important to know the catalytic activity of an enzyme and not just the concentration of the enzyme as a given amount of enzyme can have varying activity depending on reaction conditions, source of enzyme and the isolation protocol used. Also, antibodies tend to react only with structurally similar antigens. Therefore, it is often not possible to quantitate the amount of an enzyme from a given species using specific antibodies from a related species using immunoassays.

There is a need for quantitative determination of the amount of enzyme inhibitor that is present in a biological system. This is particularly important in the medical field where much attention has been directed towards the discovery of inhibitors of certain enzymes in AIDS research (Miller et al, 1989), the development of inhibitors for the control of blood pressure (Ondetti et al., 1982), or the hydrolysis of antibiotics including penicillin (Cullman, 1990). There is also an urgent demand for enzyme assays that can be automated. Automated assays are especially desired in the pharmaceutical industry, as enzymes are usually used as a target for drug discovery. Thousands of chemical compounds must be screened during the search for new drugs. The development of a new method for the assay of enzymes that is amenable to high throughput screening and automation would not only greatly facilitate such screening, but would also have many other applications and would result in markedly reduced costs for pharmaceuticals.

The pharmaceutical industry utilizes the methods mentioned above to screen compounds for discovering drugs. The process is slow due to the multiple steps required and the large amount of compounds needed to be tested. Typically, on a good day, a lab might test 100 to 1,000 compounds. However, in the race to commercialize, pharmaceutical manufacturers are facing great pressure to reduce the time required to discover new clinical drugs, cut assay costs, and screen more compounds and against more targets. Therefore, there is a very high demand to develop new methods to meet the requirements of High Throughput Screening (HTS).

There has been described a method using quenched BODIPY dye-labeled casein as a substrate for determining the activities of protease, which is sensitive and amenable to automation (Jones, L. J. et al, 1997, *Anal Biochem* 251: 144-1 52). The degree of quenching of the fluorescent tag is crucial in this method. If there is not enough quenching due to poor conjugation or degradation of the fluorescence-labeled substrate under storage, etc. the assay will not be very useful. Also this procedure has relatively high background values that reduce its sensitivity. Another example of a potentially useful high throughput assay was made by Marquardt, et al and described in PCT/WO97/43438. The assay involves many steps, including coating the wells of a microplate, washing the wells, adding biologically active substance to the wells, washing the wells once more, adding the indicator enzyme to the wells, washing the wells again and adding a color development reagent. As a result, the assay cannot be readily used in assays requiring rapid analysis.

In addition to enzymes, highly automated, screening assays for measurements of receptors, lectins, and other similar substances are also needed. For example, there has been a continuing interest in the development of simple and reliable assay procedures for β-glucanase, as this enzyme plays an important role in the depolymerization of barley β-glucan in both the brewing and the poultry production industries. Several methods have been reported for this assay including viscometry (Bourne and Pierce, 1970), reducing sugar production (Denalt et al., 1978), radial gels diffusion (Edney et al., 1986; Martin and Bamforth, 1983), and the use of azo-barley glucan (McCleary and Shameer, 1987). The detection and the quantitation of enzyme activity in finished feeds by any method developed to date is technically challenging due to the requirement for high sensitivity and the complex nature of feed itself. The development of a highly sensitive photometric method will be welcomed particularly if this could lead to a high degree of assay automation. Microtitration using microtiter plates and a microtiter plate reader would greatly facilitate such an assay.

There have been two approaches in this direction, Wirth and Wolf (1992) used a micro-plate colorimetric assay. The principle of this assay is the same as the azo-barley glucan method except the absorbency is read in microtiter plate wells. The procedure, as well as the original azo-barley glucan procedure, has the disadvantage of requiring a precipitant and a centrifuging step. It also does not have a high degree of sensitivity. Another approach has been to quantitate the amount of enzyme using the immunological properties of enzymes (Bühler, 1991; Rafael et al., 1995). The main drawback of this technique is its inability to assess the biological activity of a particular enzyme, as the immunoassay will estimate the amount of enzyme protein but not its biological activity. Also this assay would only be useful for enzymes from closely related species as antibodies tend to have high specificity.

In a review, Headon (1993) concluded that no suitable method has been reported that facilitates detection and quantitation of enzymes added to feed. This can be in part attributed to the lack of an assay that is able to detect the very low levels of enzymes that are usually added to feed.

Some viruses such as HIV and hepatitis C use proteases as part of their growth cycle. Drugs have been developed, called protease inhibitors, which treat these viral infections by blocking the function of proteases. During the past few years, protease inhibitors have been introduced as part of the drug mix in the treatment of AIDS patients (1). It is considered in most reports that protease inhibitors are the critical factors that determine the success or failure of controlling viral multiplication and disease progression (2-4). By blocking the HIV protease, these drugs prevent the processing of gag and gag-pol polyprotein precursors leading to the formation of immature non-infectious viral particles (3). The HIV protease has been isolated, cloned in *E. coli* and purified. Its minimal substrate has also been synthesized.

The following protease inhibitors are now in routine therapeutic use: amprenavir, indinavir, saquinavir, saquinavir mesylate, ritonavir, nelfinavir mesylate and lopinavir. Their number continues to grow. All are metabolized in the liver and have a relatively short half-life that requires regular administration at twice daily intervals. With the exception of ritonavir, each protease inhibitor is administered in order to achieve therapeutic levels and inhibit viral multiplication. Ritonavir, on the other hand, is administered at sub-therapeutic doses for the benefit of increasing the effective therapeutic level of any of the other protease inhibitors administered simultaneously (5). Ritonavir is useful by inhibiting the degradation in the liver of other protease inhibitors (6-8). Lopinavir has been recently approved as a protease inhibitor capsule that contains ritonavir together with another protease inhibitor.

*In vitro* studies have determined that enzyme inhibition requires a minimum level to achieve its therapeutic benefits (9), which have been established by the drug manufacturers in their clinical studies. It appears that drug levels in serum have to be higher than *in vitro* (10). It has also been determined that the HIV is able to mutate and escape from under the control of protease inhibitors, particularly when the level of the enzyme inhibitors in the serum is sub-therapeutic (5,11,12). Oftentimes the treatment failure is due to poor patient adherence, due to multiple factors (13). Treatment with much higher doses has been advocated for patients harboring the resistant mutants, although it can result in significant increase in side effects (14). Genotype and phenotype testing has become commercially available to determine the particular plasma level of protease inhibitors, which is needed to block the virus harvested from a particular patient. As a result, individualized treatment has been developed. However, for this strategy to work, the level of protease inhibitors in patient plasma has to be monitored.

Achievement of therapeutic levels by protease inhibitors are influenced by multiple factors, such as adherence to regular dosage and food intake, binding of the drugs to proteins, rate of their degradation and clearance, simultaneous treatment with other drugs as well as other as yet unknown, individual factors (1,7,9,15-17). As the result, the peak and trough level, as well as the steady state level, have large individual variability (9). For some patients, the trough can be sub-therapeutic and for others the peak can cause toxic side effects, which, in turn, reduce adherence and therapeutic benefits. Indeed, it has been determined that therapeutic benefits and drug levels are highly correlated (9). For all these reasons, clinicians have expressed the need for a test to determine the level of protease inhibitors in patients plasma (or serum).

The need to measure the levels of protease inhibitors has been fulfilled in part at specialized reference laboratories and in research or clinical/research settings mainly by a combination of HPLC/mass spectroscopy or HPLC and UV detection (4,15,18-22,22-24). The physical presence of the drugs, representing total level of protein bound and free, is detected by the HPLC based methods (4). The HPLC based methodology has high accuracy and reliability. However, in addition to being labor intensive and expensive, the timing from specimen collection to obtaining the results from the specialized laboratories could be from days to weeks, which reduces significantly their value of the results to the clinician. Indeed, when the intent is to individualize treatment, the levels have to be monitored closely by repeat testing. The test results have to be immediately followed by dose adjustment to a level indicated by the phenotype resistance testing. Otherwise, further resistance can develop during the interval of time in which the drug is not at appropriate levels.

It is of great importance in all fields and disciplines of the life sciences to utilize qualitative and quantitative analytical techniques for the detection, identification, and measurement of a wide variety of biologically important molecules. However, the commonly used methods cannot fulfill these requirements because of insufficient sensitivity (e.g. casein gel), complicated manipulations (e.g. trichloroacetic acid precipitation, centrifugation and heating), radioactive hazard (e.g. radio labeled substrates), and expensive equipment required (e.g. fluorescence polarization analyzer), etc. These procedures are usually time-consuming and often do not lend themselves to automation. Thus, the availability of a simple, sensitive, and efficient method for the assay of protease activity would be very useful for the recombinant protein industry to test intrinsic proteolytic activity, drug discovery to screen for protease inhibitors, diagnostics, and routine research.

It would therefore be useful to develop an assay method that is highly sensitive and is suitable for high throughput assays. Specifically, the assay should be suitable for a wide variety of applications, easy to use, and require only a few steps for obtaining results.

### SUMMARY OF THE INVENTION

According to the present invention, there is provided a method, kit, and assay for measuring levels of a therapeutic compound in a sample by providing a reaction vessel containing a sample, the sample includes a therapeutic compound having a biological activity and biological substance. A probe coated with a substrate for the biological substance is provided, the substrate having a detectable label. The probe is inserted into the reaction vessel such that the therapeutic compound and the substrate contact the biological substance and interact within the biological substance resulting in the label being released. The probe is removed from the reaction vessel and the quantity of detectable label is measured, thereby measuring the level of therapeutic compound. The concentration of therapeutic compound is calculated based upon a standard curve or by calculating the percentage of inhibition and expressing the results in arbitrary units.

### DESCRIPTION OF THE DRAWINGS

Other advantages of the present invention are readily appreciated as the same becomes better understood by reference to the following detailed description when considered in connection with the accompanying drawings wherein:
Figure 1 is a depiction of the assay of the present invention; and
Figures 2 (A-E) are the standard curves used with the present invention.

### DESCRIPTION OF THE PREFERRED EMBODIMENTS

Generally, the present invention provides a method, assay, and kit for the measurement of levels of therapeutic compounds in a sample. More specifically, the present invention provides a method for the qualitative and quantitative analysis of therapeutic compounds, specifically protease inhibitors.

"Ligand" as used herein refers to a bioactive molecule having specific binding affinity for another biomolecule.

By "therapeutic compounds" as used herein, the phrase is intended to include, but is not limited to, substances such as protease inhibitors, proteins, chemical substances, pharmaceuticals, drugs, medications, and any other similar treatment substances known to those of skill in the art.

"Receptor" as used herein refers to a bioactive molecule that has a specific binding affinity for another biomolecule, for example, a ligand.

"Bioactive molecule" or "biologically active substance" as used herein except where otherwise stated refers to a molecule or complex having a biological activity, for example, an enzymatic activity or binding affinity for another biomolecule.

"Probe" as used herein refers to a member arranged to be reversibly inserted into a reaction mixture. The probe can be pin-shaped, cylindrical, spire-shaped, star-shaped, cubed, or cone-shaped.

The term "label" as used herein is intended to mean any product that can be added to a compound of other item and is able to be measured. Examples of the label include, but are not limited to, fluorescent labels, luminescent labels, chromogenic molecules, radioactive tags, and other similar labels known to those of skill in the art.

The assay method of the present invention is improvement of the method (Ronald R. Marquardt, Zhibo Gan, United States Serial Number: 09/673,558) based on a one-step procedure for separating the reactants from the products (resultants) after completion of the reaction. This one-step procedure is followed by measurement of the amount of a labeled reactant or labeled product that has been left either in the reaction vessel or on the probe.

The reaction begins by the insertion of a probe that is coated with a reactant into the reaction vessel containing the same or other reactants than those coated on the probe surface. The probe surface is removed from the reaction vessel to stop the reaction. The amount of the labeled products or labeled reactant remaining in the reaction vessel or on the probe can be determined according to the intensity of its label. The amount of the label in the reaction vessel is directly or reciprocally proportional to the activity or amount of the bioactive substance that is to be measured. The method can be used for the assay of therapeutic compounds as disclosed above.

In general, the assay is carried out as follows: the quantity of labeled compound coated on the surface of the probe or the quantity of labeled substrate in the reaction vessel can first be determined by means known in the art according to the type of label used. The probe is then lowered into the reaction vessel such that the coated surface of the probe contacts the reaction mixture. The bioactive molecules present in the reaction mixture then either release label from the compound, transfers label from the substrate in the reaction mixture to the compound on the surface of the probe, or competes for binding, as described below. Once the probe has been in contact with the reaction mixture for a pre-determined period of time, the probe is removed from the reaction mixture, thereby terminating the reaction without needing to add additional chemicals. The reaction mixture is either heated or centrifuged. The quantity of label either in the reaction mixture or on the surface of the probe can then be determined, which can then be used to calculate reaction kinetics.

This is in contrast with traditional methods, which, for example, can involve providing a reaction vessel containing a reaction mixture including a bioactive molecule, adding a labeled compound that is a substrate for the bioactive molecule, stopping the reaction, that is, stopping the biological activity of the bioactive molecule, and separating unused substrate from used substrate by washing or chromatography. An alternative type of assay known in the art involves coating a reaction vessel with a compound, adding a ligand, allowing binding between the ligand and the compound to occur, washing away unbound ligand, and adding a reporter compound to detect bound ligand. These methods are less desirable than the above-described assay, as they require multiple steps and include washing and/or separation steps that can cause considerable inaccuracy in experimental results. The above-described assay does not require washing or separation steps and involves relatively few steps, resulting in shorter assay times, more reliable results, and greater ease of use.

The general principle of the non-isotopic methods of measurement of proteases and protease inhibitors in solutions has been previously described, however, for the specific uses described herein, significant modifications were made to the general method. In summary, a label, such as a fluorescent dye, is coupled to the amino-terminal with the help of a specially designed spacer to the HIV minimal substrate and is coated onto a pin with the help of a spacer. This pin is immersed into a well of a dark 96 well microtiter plate to which the enzyme was added. During the incubation, the enzyme cleaves the segment containing (a few amino-terminal residues linked to) the dye, resulting in the release of the dye into the dark well. The pin is withdrawn, which stops the reaction, and the amount of fluorescent material released is measured by a computer-controlled fluorometric reader. The release of the fluorescent material is directly proportional to the active enzyme present. Therefore, when a protease inhibitor is added during the reaction, fewer fluorescent fragments are released and less fluorescence is recorded, in a dose response manner.

Protease inhibition capacity of the serum is measured against a standard curve constructed with various dilutions of known amounts of the drug. The results are expressed in µg drug/mL of plasma.

More specifically, the method of the present invention includes the following steps. First, aliquots of plasma are collected from patients and diluted in diluent. The diluted plasma is then placed into a bath containing water heated to 100°C. It was unexpected that the drugs, which are known to be largely protein bound could still act on the protease and be measured after heating. Aliquots of diluted plasma are placed in the wells of the dark micotiter plate. In other wells serial dilutions of the standard are added in the similar manner. Also the positive and the negative controls are also diluted and added to wells. Two additional control wells are set up with diluted negative control.

Alternatively, the serum or plasma is diluted in buffer and placed in a microfiltration tube and centrifuged for 10-15 minutes. During centrifugation, the molecules that are smaller than the serum protein, including the therapeutic protease inhibitors, are forced through the filter and recovered for measurement in the assay.

The protease from the stable solution is diluted in diluent and dispensed in equal aliquots into all wells. One of the two additional control wells receive only diluent without protease. The mixtures are incubated at room temperature (18-24°C) for five minutes.

The lid is placed onto the plate and placed in the incubator at 42°C for 60 minutes. By placing the lid onto the plate, the pins become immersed into the wells, which contain the protease, with and without inhibitor. Pins are also inserted into wells that do not contain the protease and can be used to generate blank values (i.e. indicators of how much protease is released without the action of the protease). The protease cleaves the substrate and releases the dye into the well.

The reaction is stopped simultaneously in all wells by removing the lid with the pins. The dark plate is placed into a fluorometer and the fluorescence intensity is collected and transferred to the computer. The more drug that is present, the less fluorescence is expected.

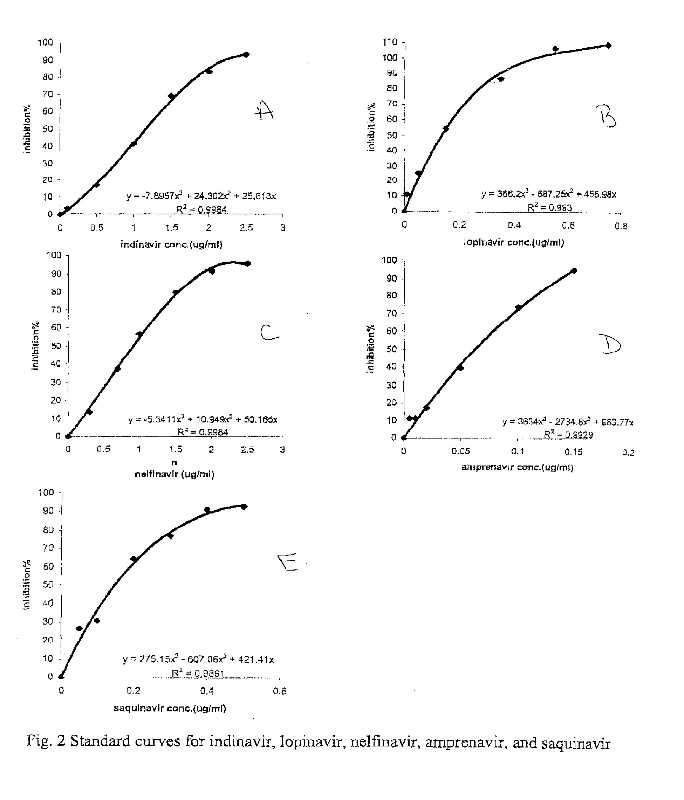

A standard curve is obtained based on the dilutions of the standard drug. The concentration of drug in the specimen is then calculated based on this standard curve, which is obtained with the same drug (Figure 2)

In an alternative embodiment, human serum is inactivated at 100°C for two main reasons: 1) to inactivate putative antibodies and other serum proteins, which can reduce the activity of the protease and 2) to kill the virus and make the test safer for the operator. When the serum is diluted most of the putative interfering proteins are also diluted to a concentration at which they no longer interfere. As the result, the test can be performed on serum that was not heat inactivated, which was completely unobvious.

An alternative calculation can also be employed wherein the quantitative relationships between all the standard curves obtained with different therapeutic compounds are established. As the result, the standard curve with only one of the therapeutic compounds is determined in the test and the standard curves for all other therapeutic compounds are generated from it by the computer, as a virtual standard curve. The level of any of the therapeutic compounds are calculated from its own virtual standard curve.

Another alternative calculation involves the use of one point standard, represented by the protease and diluted normal human serum. The percent inhibition, multiplied by the dilution factor is given as an arbitrary number of units of inhibition, with the help of comparative data.

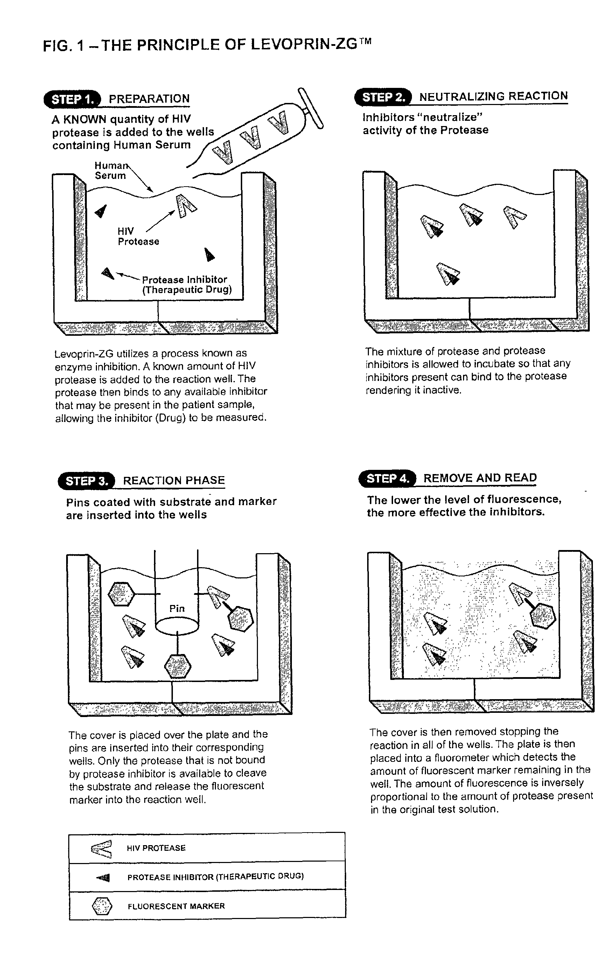

The assay itself consists of two parts, one of which is a reaction vessel and other is a probe that can fit into the reaction vessel (Figure1). The reaction vessel contains a reaction mixture that includes a therapeutic compound that is attached via a spacer specifically designed for the therapeutic compound being tested. The probe has a surface and the surface of the probe is coated with a compound. It is of note that the compound selected for coating is either a substrate for or has binding affinity for the therapeutic compound. Furthermore, the compound can be labeled, for example, fluorescently, luminescently, colorimetrically or radioactively, or the reaction mixture can contain a labeled substrate for or a labeled biomolecule having binding affinity for the bioactive molecule. In the first instance, wherein the compound is labeled, the labeled group on the compound is selected such that the activity of the therapeutic compound causes the label to be cleaved or removed from the compound and released to the reaction mixture. In the second instance, the label on the substrate within the reaction mixture is selected such that the activity of the therapeutic compound causes the label to be transferred from the labeled substrate to the compound coating the surface of the probe. Alternatively, the labeled biomolecule and the therapeutic compound in the reaction mixture can compete for binding to the compound on the surface of the probe, or the therapeutic compound in the reaction mixture can compete with the labeled compound in the reaction mixture and the compound on the surface of the probe.

Generally, the kit components include a standard, positive controls, negative controls, HIV protease in a stabilizing buffer with the sugar trehalose, microtiter plate and its lid. In the preferred embodiment a Levoprin-ZG kit is used for performing the assay. Preferably the standard is human plasma containing known concentration of the therapeutic compound, specifically a protease inhibitor. The positive controls can be aliquots of human plasma containing known amount of the therapeutic compound. The negative control can be any negative control, such as human plasma without the therapeutic compound. The diluent is preferably a buffer for dilution of protease and plasma. The lid of the microtiter has pins coated with the substrate for the protease inhibitor and the microtiter plate is preferably a dark microtiter plate.

In the process of preparing the kit, the probe, or pins in this instance, are coated with the minimal protease substrate with the help of a spacer, which maintain a critical distance between the plastic surface and the substrate. The substrate is coupled to a fluorescent dye, such as fluoreceine isothiocyanate (FITC)

The present invention is well suited for providing useful information regarding the levels of activity of molecules or substances having biological activity. Specifically, the present invention is well suited in providing information regarding protease inhibitors, and ultimately measuring the effects of drugs and other medications based on these inhibitors. The present invention determines the minimum level of enzyme inhibition needed to achieve therapeutic benefits. Thus, the present invention is useful in various genotype and phenotype testing and is well suited in analyzing and determining effective treatment of various diseases and illnesses.

The above-described assay has a wide range of applications due to its versatility. Furthermore, the assay does not require washing or "stopping" steps that are time-consuming and can lead to loss of signal or contamination. It is of note that in the examples above, the probe comprises a pin-shaped member and the reaction vessels are wells of a microtiter plate. However, it is to be understood that the probe can be of suitable shapes and arrangements, as discussed above, as can the reaction vessels.

While specific embodiments are disclosed herein, they are not exhaustive and can include other suitable designs that utilize other substances, reagents, components, structures, and devices to detect, measure, and monitor the identity, amount, and activity of molecules with biological activity. Additionally, various materials known to those of skill in the art and combinations thereof can be used to construct device of the present invention. Basically, any differing design, structure, reagents, and composite materials known to those of skill in the art can be utilized without departing from the spirit of the present invention.

Unless defined otherwise, all technical and scientific terms used herein have the same meaning as commonly understood by one of ordinary skill in the art to which the invention belongs. Although any methods and materials similar or equivalent to those described herein can be used in the practice or testing of the present invention, the preferred methods and materials are now described. All publications mentioned hereunder are incorporated herein by reference.

Throughout this application, various publications, including United States patents, are referenced by author and year. All patents are referenced by their issued patent number. Full citations for the publications are listed below. The disclosures of these publications and patents in their entireties are hereby incorporated by reference into this application in order to more fully describe the state of the art to which this invention pertains.

The invention has been described in an illustrative manner, and it is to be understood that the terminology that has been used is intended to be in the nature of words of description rather than of limitation.

Obviously, many modifications and variations of the present invention are possible in light of the above teachings. It is therefore, to be understood that within the scope of the described invention, the invention can be practiced otherwise than as specifically described.

### REFERENCES

1. Boxer M, Ellman L, Carvalho A: The Lupus Anticoagulant. Arthritis Rheum 19:1244-1251, 1976
2. Laitman RS, Glicklich D, Sablay LB, Gayzel Al, Barland P, Banks N: Effect of Long-term Normalization of Serum Complement Levels on the Course of Lupus Nephritis. Am J Med 87:132-138, 1989
3. Meyer O, Bourgeois P, Aeschlimann A: Immunoblotting Profiles in 55 Systemic Lupus Erythematosus Sera lacking Precipitating Antibodies to Extractable Nuclear Antigens. Ann Rheum Dis 48:594-599, 1989
4. Nossent JC, Huysen V, Smeenk RJT, Swaak AJG: Low Avidity Antibodies to dsDNA in Systemic Lupus Erythematosus: a Longitudinal Study of their Clinical Significance. Ann Rheum Dis 48:677-682, 1989
5. Kouri T, Crowley J, Aho K, Palosuo T, Isomäki H, Von Essen R, Heliövaara M, Carson D, Vaughan JH: Occurrence of two germline-related rheumatoid factor idiotypes in rheumatoid arthritis and in non-rheumatoid seropositive individuals. Clin Exp Immunol 82:250-256, 1990
6. Northway J, Tan E: Differentation of Anti-nuclear Antibodies Giving Speckled Staining Patterns in Immunofluoresence. Clinical Immunology and Immunopathology 1:140-148, 1972
7. Houtman P, Kallenberg CGM, Limborg T, van Rijswijk M, The T: Are Anti-nRNP Levels Specific Guides for Assessing Disease Activity in Patients with Mixed Connective Tissue Disease, Protides of the Biological Fluids. Edited by H Peters. Oxford, Pergamon Press, 1985 357
8. Mclnerey MF, Clough JD, Senitzer D, Cathcart MK: Two Distinct Subsets of Patients with Systemic Lupus Erythematosus . Clinical Immunology and Immunopathology 49:116-132, 1988
9. St.Clair EW, Burch Jr. JA, Ward MW, Keene JD, Pisetsky DS: Temporal Correlation of Antibody Responses to Different Epitopes of the Human La Autoantigen. J Clin Invest 85:515-521, 1990
10. Savige JA, Gallicchio M, Georgiou T, Davies DJ: Diverse target antigens recognized by circulating antibodies in anti-neutrophil cytoplasm antibody-associated renal vasculitides. Clin Exp Immunol 82:238-243, 1990
11. Termaat RM, Brinkman K, Nossent JC, Swaak AJG, Smeenk RJT, Berden JHM: Anti-heparan sulphate reactivity in sera from patients with systemic lupus erythematosus with renal or non-renal manifestations. Clin Exp Immunol 82:268-274, 1990
12. Grennan DM: British Society for Rheumatology Annual General Meeting Seminar Reports: Complement and Immune Complexes. British Journal of Rheumatology 28:164, 1989
13. Xu L, Chang V, Murphy A, Rock JA, Damewood M, Schlaff W, Zacur HA: Antinuclear antibodies in sera of patients with recurrent pregnancy wastage. Am J Obstet Gynecol 163:1493-1497, 1990
14. Deng J-S, Fratto J, Elenitsas R: Antinuclear matrix antibody: Hidden antinuclear antibody in patients with connective tissue diseases. Am J Clin Pathol 94:606-612, 1990
15. Misra R, Malaviya AN, Kumar R, Kumar A: Clinical relevance of the estimation of antibodies to single stranded DNA in SLE. Ind J Med Res 87:463-472, 1988
16. Tipping PG, Buchanan RRC, Riglar AG, Dimech WJ: Detection of Anti-DNA Antibodies: A Comparison Between Two Farr Assays, *Crithidia Luciliae* and a Human Chromosomal Substrate Assay. British Journal of Rheumatology 27:206-210, 1988
17. Edworthy SM, Zatarain E, McShane DJ, Bloch DA: Analysis of the 1982 ARA Lupus Criteria Data Set by Recursive Partitioning Methodology: New Insights into the Relative Merit of Individual Criteria. J Rheumatol 15:1493-1498, 1988
18. Williams REA, Mackie RM, O"Keefe R, Thomson W: The Contribution of Direct Immunoflouresence to the Diagnosis of Lupus Erythematosus. J Cut Path 16:122-125, 1989
19. Blaszczyk M, Jarzabek-Chorzelska M, Jablonska S, Chorzelski T, Kolacinska-Strasz Z, Beutner EH, Kumar V: Autoantibodies to nucleolar antigens in systemic scleroderma: Clinical correlations. Brit J Dermatol 123:421-430, 1990
20. Saiki O, Tanaka T, Kishimoto S: Defective expression of p70/75 interleukin 2 receptor in T cells from patients with systemic lupus erythematosus: A possible defect in the process of increased intracellular calcium leading to p70/75 expression. J Rheumatol 17:1303-1307, 1990
21. Suzuki Y, Kitagawa Y, Matsuoka Y, Fukuda J, Mizushima Y: Severe cerebral and systemic necrotizing vasculitis developing during pregnancy in a case of systemic lupus erythematosus. J Rheumatol 17:1408-1411, 1990
22. Tan EM: Antinuclear antibodies: diagnostic markers for autoimmune diseases and probes for cell biology. Adv Immunol 44:93-151, 1989
23. McNeil H, Simpson R, Chesterman C, Krilis S: Anti-Phospholipid Antibodies are Directed Against a Complex Antigen that Includes a Lipid-binding Inhibitor of Coagulation: _2-Glycoprotein I (apolipoprotein). Proc Natl Acad Sci USA 87:4120-4124, 1990
24. Wolf RE, Brelsford WG: Soluble Interleukin-2 Receptors in Systemic Lupus Erythematosus. Arthritis Rheum 31:729-735, 1988

## Claims

1. A method for measuring levels of a therapeutic compound in a sample, said method comprising the steps of:
providing a reaction vessel containing a sample, the sample including a therapeutic compound having a biological activity and a biological substance;
providing a probe coated with a substrate for the biological substance, the substrate having a detectable label;
inserting the probe into the reaction vessel such that the therapeutic compound and the substrate contact the biological substance and interact within the biological substance such that label is released;
removing the probe from the reaction vessel;
measuring the quantity of detectable label, thereby measuring the level of therapeutic compound; and
calculating the concentration of the therapeutic compound in the sample after the inhibitory effects of the therapeutic compound are compared to those generated by a standard curve generated with various concentrations of known amount of therapeutic compound or by calculating the percentage of inhibition and expressing the results in arbitrary units.

2. The assay according to claim 1, wherein said probe has a shape selected from the group consisting essentially of pin-shaped, cone-shaped, cubed, cylindrical, star-shaped and spire-shaped.

3. The assay according to claim 1 or claim 2, wherein said detectable label is selected from the group consisting essentially of a colorimetric label, a radioactive label, a luminescent label, and a fluorescent label.

4. The assay according to any one of claims 1 to 3, wherein said sample is a biological sample.

5. The assay according to claim 4, wherein said biological sample is human serum or plasma.

6. The assay according to claim 5, wherein said biological sample is heated to between 80 and 100°C.

7. The assay according to any preceding claim, wherein said measuring means includes standard curves generated by the same therapeutic compound or virtual standard curve generated by a different drug for comparing therapeutic compound levels to a standard.

8. The assay according to any preceding claim, wherein said biological substance is enzyme.

9. The assay according to claim 8, wherein said enzyme is protease.

10. A kit for measuring levels of a therapeutic compound in a sample, said kit comprising:
a standard;
at least one positive control;
at least one negative control;
a protease in a stabilising buffer;
a microtiter plate; and
a lid with pin for the microtiter plate.

11. The kit according to claim 10, wherein said microtiter plate is a dark microtiter plate.

12. The kit according to claim 10 or claim 11, wherein said protease in said stabilising buffer is an HIV protease.

13. The kit according to any one of claims 10 to 12, wherein said standard is human serum or plasma containing known concentration of the therapeutic compound.

14. The kit according to any one of claims 10 to 13, wherein said positive controls are aliquots of human serum or plasma containing known amount of the therapeutic compound.

15. The kit according to any one of claims 10 to 14, wherein said negative control is human serum or plasma without the therapeutic compound.

16. The kit according to any one of claims 10 to 15, wherein said therapeutic compound is a protease inhibitor.
